(19) Europäisches Patentamt
European Patent Office
Office européen des brevets

(11) **EP 4 503 049 A1**

(12) **EUROPEAN PATENT APPLICATION**
published in accordance with Art. 153(4) EPC

(43) Date of publication:
**05.02.2025  Bulletin 2025/06**

(21) Application number: 23812158.6

(22) Date of filing: **24.05.2023**

(51) International Patent Classification (IPC):
*G16H 30/40* (2018.01)    *G16H 30/20* (2018.01)
*G06N 3/08* (2023.01)    *G06N 3/042* (2023.01)
*A61B 5/055* (2006.01)

(52) Cooperative Patent Classification (CPC):
**A61B 5/055; G06N 3/04; G06N 3/042; G06N 3/08;
G16H 30/20; G16H 30/40**

(86) International application number:
**PCT/KR2023/007108**

(87) International publication number:
**WO 2023/229384 (30.11.2023 Gazette 2023/48)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **25.05.2022  KR 20220064298**

(71) Applicant: **Airs Medical Inc.**
**Seoul 08788 (KR)**

(72) Inventor: **JEONG, Keunwoo**
**Seoul 06302 (KR)**

(74) Representative: **Isarpatent**
**Patent- und Rechtsanwälte**
**Barth Hassa Peckmann & Partner mbB**
**Friedrichstraße 31**
**80801 München (DE)**

(54) **TRAINING DATA GENERATION METHOD, COMPUTER PROGRAM, AND DEVICE**

(57)    A training data generation method that is performed by a computing device including at least one processor according to an embodiment of the present disclosure includes: generating random noise corresponding to the noise of an input image; generating first noise and second noise based on the random noise; and generating a first image based on the input image and the first noise, and generating a second image based on the input image and the second noise; and the first and second images are noise-independent of each other.

[FIG. 1]

## Description

### Technical Field

The present disclosure relates to a training data generation method, computer program and device, and more particularly to a method, computer program and device for generating training data using medical images.

### Background Art

In general, a medical imaging apparatus is an apparatus that acquires a patient's body information and provides images. Such medical imaging apparatuses include X-ray imaging apparatuses, ultrasound diagnostic apparatuses, computed tomography apparatuses, and magnetic resonance imaging (MRI) apparatuses.

A magnetic resonance imaging (MRI) apparatus requires a considerable amount of time for imaging. Therefore, in the medical industry, acceleration imaging techniques to shorten MRI acquisition times are of significant importance. For accelerated MRI images to be usable in medical settings, they must include all necessary information about the subject while minimizing noise that could affect interpretation. Recently, due to this need, techniques have been developed to restore low-quality accelerated MRI images to high-quality states using artificial intelligence (AI).

To restore accelerated MRI images to high quality using AI, it is essential to effectively train the AI model. This requires not only high-quality input data but also corresponding high-quality label data. However, even if a certain level of accelerated MRI input data is obtained, there is a practical issue in that corresponding high-quality restored images are almost nonexistent. Therefore, securing the necessary training data is fundamental for building an AI model.

### Disclosure

### Technical Problem

The present disclosure is intended to overcome the problems of the prior art described above, and is directed to a training data generation method, computer program and device that generate training and label images by using only one input image.

However, objects to be achieved by the embodiments are not limited to the above-described object, and other objects may be present.

### Technical Solution

According to an embodiment of the present disclosure for achieving the above-described object, there is disclosed a training data generation method that is performed by a computing device. The training data generation method includes: generating random noise corresponding to the noise of an input image; generating first noise and second noise based on the random noise; and generating a first image based on the input image and the first noise, and generating a second image based on the input image and the second noise; and the first and second images are noise-independent of each other.

Alternatively, generating the first noise and the second noise may include generating the first noise by applying a first coefficient to the random noise and generating the second noise by applying a second coefficient, dependent on the first coefficient, to the random noise.

Alternatively, the first image may be generated by applying the first noise to the input image, and the second image may be generated by applying the second noise to the input image.

Alternatively, the training data generation method may further include, based on the first and second images, generating training and label images that are input to an artificial neural network model that outputs a high-quality medical image based on a low-quality medical image.

Alternatively, generating the training and label images may include: determining weights corresponding to the first and second images, respectively, based on a noise reduction target set in the artificial neural network model; and generating the training and label images by combining the first and second images based on the weights.

Alternatively, determining the weights may include determining weights corresponding to the first and second images, respectively, such that the sum of the weights of the first and second images is 1.

Alternatively, the input image, the first image, and the second image may each be k-space data.

Alternatively, the training data generation method may further include: generating k-space training data and k-space label data by combining the first and second images; and generating training and label images that are input to an artificial neural network model that outputs a high-quality medical image based on a low-quality medical image by performing Fourier transform on each of the k-space training data and the k-space label data.

Alternatively, generating the random noise may include: calculating the noise magnitude of the noise based on the standard deviation of pixel values that are a predetermined distance away from a center of the input image; and generating the random noise corresponding to the noise magnitude.

Alternatively, the random noise may include noise following a complex Gaussian distribution.

Alternatively, the input image, the first image, and the second image may each be a magnetic resonance image.

Alternatively, generating the random noise may include: segmenting the background of the input image, and calculating the noise magnitude based on the standard deviation of the pixel values of the background; and generating the random noise corresponding to the noise magnitude.

Alternatively, the random noise may include noise following a Rician distribution or a non-centrally chi distribution.

According to an embodiment of the present disclosure for achieving the above-described object, there is disclosed a training data generation device. The training data generation device includes: memory configured to store an input image; and a processor configured to generate random noise corresponding to the noise of the input image, generate first noise and second noise based on the random noise, generate a first image based on the input image and the first noise, and generate a second image based on the input image and the second noise, and the first image and the second image are noise-independent of each other.

Alternatively, the processor may generate the first noise and the second noise by applying a first coefficient and a second coefficient to the random noise and generate the first image and the second image by applying the first noise and the second noise to the input image, and the first coefficient and the second coefficient may be dependent on each other.

Alternatively, the processor may generate a training image and a label image for the training of an artificial neural network model by combining the first image and the second image based on a noise reduction goal target set in the artificial neural network model, and the artificial neural network model may trained to output a high-quality medical image based on a low-quality medical image.

According to an embodiment of the present disclosure for achieving the above-described object, there is disclosed a computer program stored in a computer-readable storage medium. The computer program performs operations of generating training data when executed on at least one processor. In this case, the operations include operations of: generating random noise corresponding to the noise of an input image; generating first noise and second noise based on the random noise; and generating a first image based on the input image and the first noise, and generating a second image based on the input image and the second noise, and the first and second images are noise-independent of each other.

## Advantageous Effects

According to the solution of the present disclosure described above, the present disclosure may generate training and label images by using one input image, and may train an artificial neural network even without a high-quality label image.

Furthermore, according to the solution of the present disclosure described above, the present disclosure may generate training and label images based on the noise reduction target of an artificial neural network model, so that the noise reduction target of the artificial neural network model can be adjusted quantitatively and finely.

## Description of Drawings

FIG. 1 is a block diagram of a computing device according to an embodiment of the present disclosure;

FIG. 2 is a block diagram of an artificial neural network model training system according to an embodiment of the present disclosure;

FIG. 3 is a flowchart showing a method of operating a training data generation device according to an embodiment of the present disclosure;

FIG. 4 is a flowchart showing a method of operating a training data generation device according to an embodiment of the present disclosure;

FIG. 5 is a flowchart showing a method of operating an artificial neural network model according to an embodiment of the present disclosure; and

FIG. 6 is a block diagram of an artificial neural network model training system according to an embodiment of the present disclosure.

**Mode for Invention**

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

In the present specification, the 'image' may refer to multi-dimensional data composed of discrete image elements (e.g., pixels in a 2D image or voxels in a 3D image). For example, an image may include medical images acquired by medical imaging machines such as a magnetic resonance imaging (MRI) machine, a computed tomography (CT) scanner, an ultrasonic scanner, and an X-ray machine.

The term "medical image" used herein is a concept collectively referring to all forms of images encompassing medical knowledge, and may include images acquired through various modalities such as visible light cameras, IR cameras, ultrasound, X-ray, CT, MRI, and PET.

The term "medical image archiving and communication system (PACS)" used herein refers to a system that stores, processes, and transmits medical images in accordance with the Digital Imaging and Communications in Medicine (DICOM) standard. For example, the "PACS" operates in conjunction with digital medical imaging equipment, and stores medical images such as magnetic resonance imaging (MRI) images and computed tomography (CT) images in accordance with the DICOM standard. The "PACS" may transmit medical images to terminals inside and outside a hospital over a communication network. In this case, meta information such as reading results and medical records may be added to the medical images.

In the present specification, the 'object' is a target for imaging, and may include a human, an animal, or a part thereof. For example, an object may include a part (organ) of the body or a phantom. The phantom refers to a volume material having a density and an effective atomic number considerably close to those of an organism, and may include a spherical phantom having properties similar to those of the body.

A magnetic resonance image (MRI) system is a system that acquires images of tomographic areas of an object by representing the intensity of a magnetic resonance (MR) signal for a radio frequency (RF) signal generated from a magnetic field having a specific intensity in the form of contrasts between light and darkness.

The MRI system allows a main magnet to form a static magnetic field, and aligns the magnetic dipole moment direction of a specific atomic nucleus of an object, located in the static magnetic field, in the direction of the static magnetic field. A gradient magnetic field coil may generate a gradient magnetic field by applying a gradient signal to the static magnetic field, thereby inducing a different resonance frequency for each portion of the object. An RF coil may radiate a magnetic resonance signal in accordance with the resonance frequency of a portion where an image is to be acquired. Furthermore, as the gradient magnetic field is formed, the RF coil may receive magnetic resonance signals of different resonance frequencies radiated from various portions of the object. The MRI system acquires an image by applying an image reconstruction technique to the magnetic resonance signals received through this step. In addition, the MRI system may reconstruct a plurality of magnetic resonance signals into image data by performing serial or parallel signal processing on the plurality of magnetic resonance signals received by multi-channel RF coils.

The foregoing descriptions of the terms are intended to help to understand the present disclosure. Accordingly, it should be noted that unless the above-described terms are explicitly described as limiting the content of the present disclosure, they should not be interpreted as limiting the technical spirit of the present disclosure.

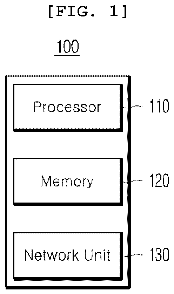

FIG. 1 is a block diagram of a computing device according to an embodiment of the present disclosure.

A computing device 100 according to an embodiment of the present disclosure may be a hardware device or part of a hardware device that performs the comprehensive processing and calculation of data, or may be a software-based computing environment that is connected to a communication network. For example, the computing device 100 may be a server that performs an intensive data processing function and shares resources, or may be a client that shares resources through interaction with a server. Furthermore, the computing device 100 may be a cloud system in which a plurality of servers and clients interact with each other and comprehensively process data. Since the above descriptions are only examples related to the type of computing device 100, the type of computing device 100 may be configured in various manners within a range understandable to those skilled in the art based on the content of the present disclosure.

Referring to FIG. 1, the computing device 100 according to an embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to an embodiment of the present disclosure may be understood as a configuration unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

According to an embodiment of the present disclosure, a processor 110 may generate training data that is input to an artificial neural network model. The artificial neural network model is trained using training data, and may output a low-quality image as a high-quality image. For example, the artificial neural network model may generate a high-quality image by removing noise from an input low-quality image.

The artificial neural network model may include at least one neural network. The neural network may include network models such as a Deep Neural Network (DNN), a Recurrent Neural Network (RNN), a Bidirectional Recurrent Deep Neural Network (BRDNN), a Multilayer Perceptron (MLP), and a Convolutional Neural Network (CNN), but is not limited thereto.

The image may be a medical image, e.g., a magnetic resonance image. In this case, the magnetic resonance image may be acquired through accelerated imaging. The accelerated imaging may be understood as imaging techniques that shortens imaging time by increasing the acceleration factor compared to regular imaging. The acceleration factor is a term used in a parallel imaging technique, and may be understood as a value obtained by dividing the number of signal lines fully sampled in the k-space domain by the number of signal lines sampled through imaging.

The processor 110 may generate training data based on one input image. The input images may be medical images, magnetic resonance images, or k-space data. The training data may include at least one training image and a label image corresponding to the training image. The label image may have higher quality than the training image. Alternatively, the training image and the label image may be noise-independent of each other. More specifically, the training image and the label image may be noise-independent of each other by a preset value, and may be noise-dependent on each other by a preset value. In this case, the artificial neural network model trained with the training and label images may be trained to remove noise by a preset value.

According to an embodiment of the present disclosure, the processor 110 may generate two images noise-independent of each other by using one input image and generate training data by using the two images. Accordingly, an artificial neural network may be trained without a high-quality labeled image. Furthermore, the noise reduction target of the artificial neural network model may be adjusted in detail by setting the degree of noise independence or noise dependence of the two images.

According to an embodiment of the present disclosure, the processor 110 may train an artificial neural network model by using training and label images generated based on one input image.

According to an embodiment of the present disclosure, the processor 110 may train the artificial neural network model to improve image quality. The image may be, for example, a magnetic resonance image, or may be acquired by accelerated imaging. The accelerated imaging may be understood as an imaging technique that shortens imaging time by increasing the acceleration factor compared to regular imaging. The acceleration factor is a term used in a parallel imaging technique, and may be understood as a value obtained by dividing the number of signal lines fully sampled in the K-space domain by the number of signal lines sampled through imaging. For example, an acceleration factor of 2 can be understood as obtaining a number of signal lines equivalent to half of the number of fully sampled signal lines when lines are obtained by sampling a magnetic resonance signal in the phase encoding direction. Accordingly, as the acceleration factor increases, the imaging time of a magnetic resonance image may decrease proportionally. That is, when the acceleration factor is increased during the imaging of a magnetic resonance image, accelerated imaging in which magnetic resonance image imaging time is shortened may be implemented.

When necessary, the processor 110 may generate a user interface that provides an environment for interaction with the user of the computing device 100 or the user of any client. For example, the processor 110 may generate a user interface that receives one or more input images and the noise reduction target of the artificial neural network model.

The processor 110 may generate a user interface that allows functions such as the output, modification, change, and/or addition of data to be implemented based on an external input signal applied from a user. Since the role of the user interface described above is merely an example, the role of the user interface may be defined in various manners within a range understandable to those skilled in the art based on the content of the present disclosure.

The memory 120 according to an embodiment of the present disclosure may be understood as a configuration unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data required for the processor 110 to perform operations, a combination of data, and program code executable on the processor 110. For example, the memory 120 may store one or more input images received through the network unit 130, which will be described later. Furthermore, the memory 120 may store training and label images generated from one or more input images by the processor 110. The memory 120 may store a noise reduction target set in the artificial neural network model. Furthermore, the memory 120 may store program code that operates the processor 110 to generate training data.

The network unit 130 according to an embodiment of the present disclosure may be understood as a configuration unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data required for the processor 110 to perform operations through wired or wireless communication with any system or client. Furthermore, the network unit 130 may transmit data generated through the operation of the processor 110 through wired or wireless communication with any system or any client. For example, the network unit 130 may receive medical images through communication with a PACS, a cloud server for performing tasks such as the improvement of the resolution of medical images and the standardization of medical images, or the computing device 100. The network unit 130 may transmit improved images output from an artificial intelligence model and a mathematical model through communication with the above-described system, server, or computing device 100.

When necessary, the network unit 130 may provide the user interface, generated by the processor 110, to any system or client through wired or wireless communication with the system or client. For example, the network unit 130 may provide a user interface for visualizing data, processed by the processor 110, through communication with a PACS or the computing device 100 including a display to the system or device. The network unit 130 may receive an external input signal, applied from a user, through a user interface from the above-described system or device, and transmit it to the processor 110. In this case, the processor 110 may operate to implement functions such as the output, modification, change, or addition of data based on the external input signal transmitted from the network unit 130. Meanwhile, even without communication through the network unit 130, the system or device provided with a user interface may operate on its own to implement functions such as the output, modification, change, or addition of data according to an external input signal applied from the user.

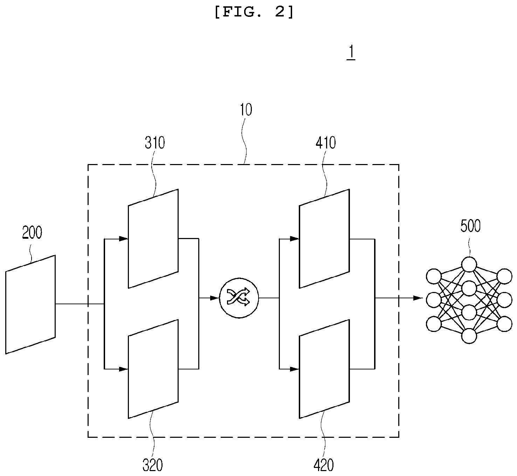

FIG. 2 is a block diagram of an artificial neural network model training system according to an embodiment of the present disclosure.

Referring to FIG. 2, the artificial neural network model training system 1 includes a training data generation device 10 and an artificial neural network model 500. The training data generation device 10 receives one input image 200 and generates training data including a training image 410 and a label image 420. The artificial neural network model 500 is trained to output a high-quality image based on a low-quality image by using the training data.

The training data generation device 10 may measure the noise magnitude of the input image 200. The noise magnitude may refer to the standard deviation of pixel values included in the input image 200. For example, when the input image 200 is a magnetic resonance image, the background of the input image 200 is segmented, and this refers to the standard deviation of pixel values of the segmented background.

The training data generation device 10 may generate random noise having the magnitude that is the same as the measured noise magnitude. The random noise may mean noise that follows a complex Gaussian distribution, a Rician distribution, or a noncentral chi distribution. Furthermore, the random noise may be an image that has the same magnitude as the input image. In this case, the per-pixel noise of the random noise, which is in the form of an image, may be generated independently.

Meanwhile, the noise magnitude may be set for each pixel of the input image 200. Additionally, the noise magnitude may be set to be proportional to a structure factor (g-factor) value.

The training data generation device 10 generates first noise by multiplying the random noise by a first coefficient, and generates second noise by multiplying the random noise by a second coefficient. In this case, once any one of the first and second coefficients has been determined, the other value may be determined.

The training data generation device 10 generates a first image 310 based on the input image 200 and the first noise, and generates a second image 320 based on the input image 200 and the second noise. For example, the training data generation device 10 generates the first image 310 by adding the first noise to the input image 200, and generates the second image 320 by adding the second noise to the input image 200.

In this case, the first and second coefficients are determined such that the first and second images are noise-independent of each other. That is, the first and second coefficients are determined such that the noise of the input image 200 plus the first noise and the noise of the input image 200 plus the second noise are noise-independent of each other.

The training data generation device 10 generates the training image 410 and the label image 420 by combining the first image 310 and the second image 320. Methods of combining first and second images to generate training and label images may be various. For example, the training data generation device 10 determines weights corresponding to the first image 310 and the second image 320, respectively, based on the noise reduction target set in the artificial neural network model 500. For example, the weights refer to the coefficients of linear combination of the first and second images 310 and 320. The training and label images 410 and 420 may be generated according to, for example, Equation 1 below:

$$T = X$$

$$L = \frac{1}{a} * X + \left(1 - \frac{1}{a}\right) * Y \qquad (1)$$

In Equation 1, T denotes the learning image 410, L denotes the label image 420, $X$ denotes the first image 310, and $Y$ denotes the second image 320. $a$ means training the artificial neural network model 500 to achieve a noise reduction target, e.g., to reduce noise by *a* times or increase the signal-to-noise ratio (SNR) by *a* times.

In this case, the signal sizes of the training image 410 and the label image 420 are the same, and the label image 420 includes noise dependent on the noise of the training image 410 by $\frac{1}{a}$ .Since the artificial neural network model 500 does not learn independent noise, it is trained to increase the SNR by *a* times. For example, when *a* is infinite, the training image 410 is the first image 310 and the label image 420 is the second image 320. In this case, the first image 310 and the second image 320 are noise-independent of each other, so that the artificial neural network model 500 is trained to increase the SNR infinitely. Meanwhile, Equation 1 is an example, and a method of generating the training and label images 410 and 420 by combining the first and second images 310 and 320 is not limited thereto.

The training data generation device 10 may provide training data including the generated training image 410 and label image 420 to the artificial neural network model 500.

Meanwhile, the configuration shown in FIG. 1 is illustrative. The training data generation device 10 and the artificial neural network model 500 may be included in different systems, respectively. In this case, the training data generation device 10 and the artificial neural network model 500 may transmit and receive training data over a network.

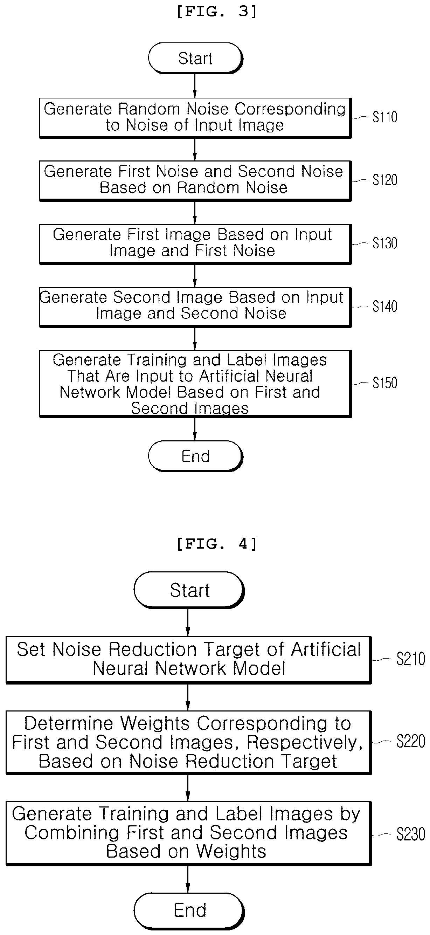

FIG. 3 is a flowchart showing a method of operating a training data generation device according to an embodiment of the present disclosure.

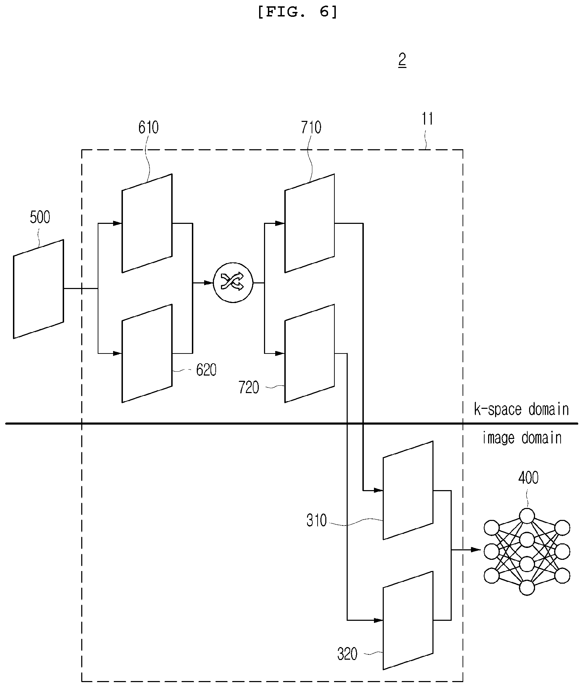

Referring to FIG. 3, the training data generation device may be an embodiment of the training data generation device 10 of FIG. 1 or the training data generation device 11 of FIG. 6.

The training data generation device generates random noise corresponding to the noise of the input image in step S110. More specifically, random noise having the magnitude that is the same as the noise magnitude of the input image is generated.

When the input image is k-space data, the training data generation device calculates the noise magnitude based on the standard deviation of pixel values that are a predetermined distance away from the center of the input image. Then, the noise that follows a complex Gaussian distribution is generated using the calculated magnitude.

When the input image is a magnetic resonance image, the training data generation device segments the background of the input image and calculates the noise magnitude based on the standard deviation of pixel values of the background. Then, the noise that follows a Rician distribution or a noncentral chi distribution is generated using the calculated magnitude.

The training data generation device generates first noise and second noise based on the random noise in step S120. In this case, the first noise is generated by applying the first coefficient to the random noise, the second noise is generated by applying the second coefficient to the random noise, and the second coefficient is determined based on a preset value and the first coefficient.

The first and second coefficients are determined such that the noise of the input image plus the first noise and the noise of the input image plus the second noise are noise-independent of each other. That is, the first and second coefficients are dependent on each other. For example, the training data generation device generates the first noise by multiplying the random noise by the first coefficient, and generates the second noise by multiplying the random noise by the second coefficient.

The training data generation device generates the first image based on the input image and the first noise in step S130, and generates the second image based on the input image and the second noise in step S140. The sequential positions of steps S130 and S140 are not limited thereto, and steps S130 and S140 may be performed simultaneously. In this case, the first image is generated by adding the first noise to the input image, and the second image is generated by adding the second noise to the input image. The first and second images are noise-independent of each other.

The training data generation device generates a training image and a label image to be input to the artificial neural network model based on the first and second images in step S150. In this case, the preprocessing of the first and second images may be performed. For example, an operation of converting the domain of the first and second images may be performed. Meanwhile, step S150 is not necessarily performed, and may be omitted.

FIG. 4 is a flowchart showing a method of operating a training data generation device according to an embodiment of the present disclosure.

Referring to FIG. 4, the training data generation device may be an embodiment of the training data generation device 10 of FIG. 1 or the training data generation device 11 of FIG. 6.

The training data generation device may set the noise reduction target of the artificial neural network model in step S210.

The training data generation device determines weights corresponding to the first and second images based on the noise reduction target in step S220. For example, the training data generation device may cause the sum of the weight of the first image and the weight of the second image to be 1.

The training data generation device generates a learning image and a label image by combining the first and second images based on the weights in step S230.

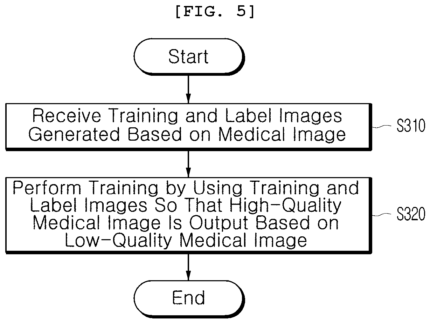

FIG. 5 is a flowchart showing a method of operating an artificial neural network model according to an embodiment of the present disclosure.

Referring to FIG. 5, the artificial neural network model may be an embodiment of the artificial neural network model 500 of FIG. 1 or 6.

The artificial neural network model receives the training and label images each generated based on medical image in step S310. In this case, the training and label images are generated by combining the first and second images generated based on the medical image, and the first and second images are noise-independent of each other. The first image is generated based on the medical image and the first noise, and the second image is generated based on the medical image and the second noise. The first noise and the second noise are generated based on the random noise having the magnitude that is the same as the noise magnitude of the medical image.

The artificial neural network model is trained to output a high-quality medical image based on a low-quality medical image by using the training and label images in step S320.

FIG. 6 is a block diagram of an artificial neural network model training system according to an embodiment of the present disclosure.

Referring to FIG. 6, the artificial neural network model training system 2 is similar to the artificial neural network model training system 1 of FIG. 2, so that descriptions of common points are omitted. A training image 410 and a label image 420 may be magnetic resonance images in the image domain. One or more input images 600 may be images of the k-space domain, i.e., k-space data.

The training data generation device 11 measures the noise magnitude of the input image 600. The noise magnitude refers to the standard deviation of pixel values that are a predetermined distance away from the center of the input image 600. The training data generation device 11 may generate random noise having the magnitude that is the same as the measured noise magnitude. The random noise may mean the noise that follows a complex Gaussian distribution.

The training data generation device 11 generates first noise by multiplying the random noise by a first coefficient, and generates second noise by multiplying the random noise by a second coefficient. The training data generation device 11 generates a first image 710 based on the input image 600 and the first noise, and generates a second image 720 based on the input image 600 and the second noise. The first and second images 710 and 720 are noise-independent of each other.

The training data generation device 11 generates k-space training data 810 and k-space label data 820 based on the first and second images 710 and 720. Since this is similar to the method described above in conjunction with Equation 1 of FIG. 2 and FIG. 4, the description thereof will be omitted below. The training data generation device 11 may generate the training and label images 410 and 420 by performing Fourier transform on each of the k-space training and label data 810 and 820, and may provide the training and label images 410 and 420 to the artificial neural network model 500.

The foregoing description of the present disclosure is intended for illustrative purposes, and a person having ordinary skill in the art to which the present disclosure pertains will understand that modifications into other specific forms may be easily performed without departing from the technical spirit or essential features of the present disclosure. Therefore, it should be understood that the embodiments described above are all illustrative but not restrictive in all respects. For example, each component described as single may be implemented in a distributed manner, and similarly, components described as distributed may also be implemented in a combined form.

The scope of the present disclosure is defined by the attached claims rather than the detailed description above, and all variations and/or modifications derived from the meanings and scope of the attached claims and their equivalent concepts should be construed as being included in the scope of the present disclosure.

**Claims**

1. A training data generation method, the training data generation method being performed by a computing device including at least one processor, the training data generation method comprising:

    generating random noise corresponding to noise of an input image;
    generating first noise and second noise based on the random noise; and
    generating a first image based on the input image and the first noise, and generating a second image based on the input image and the second noise;
    wherein the first and second images are noise-independent of each other.

2. The training data generation method of claim 1, wherein generating the first noise and the second noise comprises generating the first noise by applying a first coefficient to the random noise and generating the second noise by applying a second coefficient, dependent on the first coefficient, to the random noise.

3. The training data generation method of claim 1, wherein:

   the first image is generated by applying the first noise to the input image; and
   the second image is generated by applying the second noise to the input image.

4. The training data generation method of claim 1, further comprising, based on the first and second images, generating training and label images that are input to an artificial neural network model that outputs a high-quality medical image based on a low-quality medical image.

5. The training data generation method of claim 4, wherein generating the training and label images comprises:

   determining weights corresponding to the first and second images, respectively, based on a noise reduction target set in the artificial neural network model; and
   generating the training and label images by combining the first and second images based on the weights.

6. The training data generation method of claim 5, wherein determining the weights comprises determining weights corresponding to the first and second images, respectively, such that a sum of the weights of the first and second images is 1.

7. The training data generation method of claim 1, wherein the input image, the first image, and the second image are each k-space data.

8. The training data generation method of claim 7, further comprising:

   generating k-space training data and k-space label data by combining the first and second images; and
   generating training and label images that are input to an artificial neural network model that outputs a high-quality medical image based on a low-quality medical image by performing Fourier transform on each of the k-space training data and the k-space label data.

9. The training data generation method of claim 7, wherein generating the random noise comprises:

   calculating noise magnitude of the noise based on a standard deviation of pixel values that are a predetermined distance away from a center of the input image; and
   generating the random noise corresponding to the noise magnitude.

10. The training data generation method of claim 7, wherein the random noise comprises noise following a complex Gaussian distribution.

11. The training data generation method of claim 1, wherein the input image, the first image, and the second image are each a magnetic resonance image.

12. The training data generation method of claim 11, wherein generating the random noise comprises:

    Segmenting a background of the input image, and calculating the noise magnitude based on a standard deviation of pixel values of the background; and
    generating the random noise corresponding to the noise magnitude.

13. The training data generation method of claim 11, wherein the random noise comprises noise following a Rician distribution or a noncentral chi distribution.

14. A training data generation device, comprising:

    memory configured to store an input image; and
    a processor configured to generate random noise corresponding to noise of the input image, generate first noise and second noise based on the random noise, generate a first image based on the input image and the first noise, and generate a second image based on the input image and the second noise;
    wherein the first image and the second image are noise-independent of each other.

**15.** The training data generation device of claim 13, wherein:

the processor generates the first noise and the second noise by applying a first coefficient and a second coefficient to the random noise, and generates the first image and the second image by applying the first noise and the second noise to the input image; and
the first coefficient and the second coefficient are dependent on each other.

**16.** The training data generation device of claim 13, wherein:

the processor generates a training image and a label image for training of an artificial neural network model by combining the first image and the second image based on a noise reduction goal target set in the artificial neural network model; and
the artificial neural network model is trained to output a high-quality medical image based on a low-quality medical image.

**17.** A computer program stored in a computer-readable storage medium, the computer program performing operations of generating training data when executed on at least one processor,

wherein the operations comprise operations of:

generating random noise corresponding to noise of an input image;
generating first noise and second noise based on the random noise; and
generating a first image based on the input image and the first noise, and generating a second image based on the input image and the second noise; and

wherein the first and second images are noise-independent of each other.

[FIG. 1]

100

Processor — 110

Memory — 120

Network Unit — 130

[FIG. 2]

[FIG. 3]

Start

Generate Random Noise Corresponding
to Noise of Input Image — S110

Generate First Noise and Second Noise
Based on Random Noise — S120

Generate First Image Based on Input
Image and First Noise — S130

Generate Second Image Based on Input
Image and Second Noise — S140

Generate Training and Label Images
That Are Input to Artificial Neural
Network Model Based on First and
Second Images — S150

End

[FIG. 4]

Start

Set Noise Reduction Target of Artificial
Neural Network Model — S210

Determine Weights Corresponding to
First and Second Images, Respectively,
Based on Noise Reduction Target — S220

Generate Training and Label Images by
Combining First and Second Images
Based on Weights — S230

End

[FIG. 5]

```
                    ┌─────────────┐
                    │    Start     │
                    └─────────────┘
                           │
                           ▼
      ┌───────────────────────────────────────┐
      │   Receive Training and Label Images    │ ── S310
      │   Generated Based on Medical Image     │
      └───────────────────────────────────────┘
                           │
                           ▼
      ┌───────────────────────────────────────┐
      │  Perform Training by Using Training and │
      │  Label Images So That High-Quality     │ ── S320
      │  Medical Image Is Output Based on      │
      │  Low-Quality Medical Image             │
      └───────────────────────────────────────┘
                           │
                           ▼
                    ┌─────────────┐
                    │     End      │
                    └─────────────┘
```

[FIG. 6]

<table>
<tr><td colspan="2" align="center">**INTERNATIONAL SEARCH REPORT**</td><td>International application No.<br><br>**PCT/KR2023/007108**</td></tr>
</table>

**A.    CLASSIFICATION OF SUBJECT MATTER**

**G16H 30/40**(2018.01)i; **G16H 30/20**(2018.01)i; **G06N 3/08**(2006.01)i; **G06N 3/042**(2023.01)i; **A61B 5/055**(2006.01)i

According to International Patent Classification (IPC) or to both national classification and IPC

**B.    FIELDS SEARCHED**

Minimum documentation searched (classification system followed by classification symbols)

G16H 30/40(2018.01); G06N 3/04(2006.01); G06N 3/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 노이즈(noise), 이미지(image), 독립(independent), 라벨(label), 학습(learning)

**C.    DOCUMENTS CONSIDERED TO BE RELEVANT**

| Category* | Citation of document, with indication, where appropriate, of the relevant passages | Relevant to claim No. |
|---|---|---|
| X<br>Y | DESAI, Arjun D. et al. Noise2Recon: A Semi-Supervised Framework for Joint MRI Reconstruction and Denoising. 30 September 2021, pp. 1-17.<br>          See pages 1, 3-5, 9 and 15. | 1-12,14-17<br>13 |
| Y | VARADARAJAN, Divya et al. A Majorize-Minimize Framework for Rician and Non-Central Chi MR Images. IEEE TRANSACTIONS ON MEDICAL IMAGING. VOL. 34, NO. 10, October 2015, pp. 2191-2202.<br>          See pages 2191-2193. | 13 |
| A | TIAN, Qiyuan et al. SDnDTI: Self-supervised deep learning-based denoising for diffusion tensor MRI. NeuroImage. 253 (2022), 119033, 01 March 2022, pp. 1-17.<br>          See pages 1-17. | 1-17 |
| A | SONG, Hwanjun et al. Learning from Noisy Labels with Deep Neural Networks: A Survey. IEEE TRANSACTIONS ON NEURAL NETWORKS AND LEARNING SYSTEMS. 10 March 2022, pp. 1-19.<br>          See pages 1-19. | 1-17 |

☑ Further documents are listed in the continuation of Box C.        ☑ See patent family annex.

| | |
|---|---|
| *        Special categories of cited documents:<br>"A"    document defining the general state of the art which is not considered to be of particular relevance<br>"D"    document cited by the applicant in the international application<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **30 August 2023** | **30 August 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| | International application No. |
|---|---|
| | **PCT/KR2023/007108** |

**C. DOCUMENTS CONSIDERED TO BE RELEVANT**

| Category* | Citation of document, with indication, where appropriate, of the relevant passages | Relevant to claim No. |
|---|---|---|
| PX | KR 10-2513218 B1 (AIRS MEDICAL INC.) 25 April 2023 (2023-04-25)<br>See paragraphs [0063]-[0075]; claims 1-17; and figure 2.<br>(This document is a published earlier application that serves as a basis for claiming priority of the present international application.) | 1-17 |

Form PCT/ISA/210 (second sheet) (July 2022)

INTERNATIONAL SEARCH REPORT
**Information on patent family members**

International application No.

**PCT/KR2023/007108**

| Patent document cited in search report | Publication date (day/month/year) | Patent family member(s) | Publication date (day/month/year) |
|---|---|---|---|
| KR 10-2513218 B1 | 25 April 2023 | None | |

Form PCT/ISA/210 (patent family annex) (July 2022)